# EUROPÄISCHE PATENTSCHRIFT

(11) **EP 3 363 481 B1**
(45) Veröffentlichungstag und Bekanntmachung des Hinweises auf die Patenterteilung: **13.05.2020**
(21) Anmeldenummer: 17156203.6
(22) Anmeldetag: 15.02.2017
(51) Int. Cl.: A61M 1/16

(54) **HÄMODIALYSEANORDNUNG**
HEMODIALYSIS ASSEMBLY
SYSTÈME D'HÉMODIALYSE

(43) Veröffentlichungstag der Anmeldung: 22.08.2018
(73) Patentinhaber: D.Med Consulting AG, 40468 Düsseldorf (DE)
(72) Erfinder: Breuch, Gerd, 20249 Hamburg (DE); Biermann, Frank, 22455 Hamburg (DE); Breuch, Julius, 20457 Hamburg (DE); Holzschuh, Robert, 22301 Hamburg (DE)
(74) Vertreter: terpatent Patentanwälte ter Smitten Eberlein-Van Hoof Rütten Daubert

(56) Entgegenhaltungen:
- AT-U1- 4 009
- US-A- 5 334 139
- US-A- 5 861 555
- US-A1- 2012 216 903
- US-B2- 8 789 558

## Beschreibung

Die Erfindung bezieht sich auf eine Hämodialyseanordnung mit einer Dialysewasser-Zulaufleitung und einer Dialysat-Ablaufleitung.

Durch die Dialysewasser-Zulaufleitung der Hämodialyseanordnung fließt Dialysewasser aus einer Dialysewasser-Quelle zu einem Dialysegerät, in dem das Dialysewasser durch Lösen bzw. Vermischen mit festen oder flüssigen Substanzen zur Dialyseflüssigkeit gemacht wird. Die Dialyseflüssigkeit wird zu einem Dialysator gepumpt, in dem harnpflichtigen Substanzen aus dem Patientenblut in die Dialyseflüssigkeit übertreten, die auf diese Weise begrifflich zu einem Dialysat bzw. zu einer zur Dialysatflüssigkeit wird. Von dem Dialysator fließt das Dialysat schließlich durch eine Dialysat-Ablaufleitung von dem Dialysegerät zu einem Abwasser-Ziel.

Die Dialysewasser-Quelle kann beispielsweise eine stationäre Aufbereitungsanlage sein, die aus Trinkwasser aus dem öffentlichen Wassernetz durch entsprechende Filtrierung Dialysewasser generiert. Die Dialysewasser-Quelle kann alternativ auch ein austauschbarer Dialysewasser- Tank sein. Das von der Dialysewasser-Quelle kommende Dialysewasser hat in der Regel eine Temperatur von 10°C bis 20°C. Die Dialyseflüssigkeit wird in dem Dialysegerät stromaufwärts des Dialysators auf ca. 37 °C aufgeheizt, damit die Dialyseflüssigkeit in dem Dialysator dem Patientenblut keine Wärme entzieht. Die schließlich aus dem Dialysegerät abfließende Dialysatflüssigkeit hat daher stets eine Temperatur von knapp 37°C. Bei einer Dialysebehandlung werden 100-300 I Dialysewasser verbraucht, das von einer Liefertemperatur von 10-20°C auf eine Arbeitstemperatur von mindestens 37 °C erwärmt werden muss, was bezüglich der hierfür erforderlichen Energie einen nennenswerten Kostenfaktor darstellt.

Aus US 3 878 095 A und US 5 861 555 A ist jeweils eine Hämodialyseanordnung bekannt, die einen gräteinternen Wärmetauscher aufweist, in dem die Wärme der abfließenden Dialysatflüssigkeit teilweise auf die in den Wärmetauscher einfließende Dialyseflüssigkeit übertragen wird. Ein derartiger geräteseitiger Wärmetauscher ist verhältnismäßig aufwändig und kann bei älteren Hämodialyseanordnungen bzw. Dialysegeräten, die keinen derartigen Wärmetauscher aufweisen, nicht ohne weiteres nachgerüstet werden.

Aus EP 2 497 507 B1 ist ein Dialysegerät bekannt, das eine Wärmepumpe aufweist, um die Wärme der abfließenden Dialysatflüssigkeit auf die zuströmende Dialyseflüssigkeit zu übertragen. Eine Wärmetauscherpumpe ist operativ noch erheblich aufwändiger als ein einfacher Wärmetauscher, wobei eine Nachrüstung einer Wärmepumpe in einem Dialysegerät praktisch ausgeschlossen ist.

Aus US 2012/0216903 A1 und US 8 789 558 B2 sind jeweils zusammengefasste Doppel-Leitungen für Flüssigkeiten bekannt.

Aufgabe der Erfindung ist es demgegenüber, eine Hämodialyseanordnung mit einem einfachen Wärmetauscher zu schaffen.

Diese Aufgabe wird erfindungsgemäß gelöst mit einer Hämodialyseanordnung mit den Merkmalen des Anspruchs 1.

Die erfindungsgemäße Hämodialyseanordnung weist mindestens eine Dialysewasser-Zulaufleitung und eine Dialysat-Ablaufleitung auf. Beide Leitungen sind in der Dialysepraxis häufig relativ lang, und sind daher gut dazu geeignet, parallel zueinander und benachbart zueinander in einem entsprechenden Hohlraum einer sich entlang der Flussachse erstreckenden Wärmetauschermanschette im Gegenstrom und aneinander anliegend angeordnet zu sein. Die Wärmetauschermanschette sorgt zum Einen dafür, dass die beiden Leitungen im Gegenstrom zueinander angeordnet und über einen großen Teil ihrer Gesamtlänge unmittelbar aneinander angrenzend fixiert sind. Hierdurch wird ein Wärmeaustausch zwischen der Zulaufleitung und der Ablaufleitung begünstigt. Ferner gewährt die Wärmetauschermanschette auch eine gewisse Wärmeisolation, so dass der Wärmeverlust der beiden Leitungen im Bereich der Wärmetauschermanschette reduziert ist.

Die Wärmetauschermanschette kann nachrüstbar ausgebildet sein, oder aber kann alternativ serienmäßig mit der Zulaufleitung und der Ablaufleitung als betriebsbereites Set angeboten werden. Letzteres ist insbesondere dann sinnvoll, wenn die Zulaufleitung und die Ablaufleitung Einmalartikel sind, die für jede Dialysebehandlung durch eine neue Zulaufleitung und Ablaufleitung ersetzt werden.

Die die Zulaufleitung und die Ablaufleitung im Gegenstrom zueinander fixierende Wärmetauschermanschette stellt ein sehr einfaches und damit preiswertes Mittel dar, mit dem ohne weiteren Primärenergie- Verbrauch das Dialysewasser auf dem Weg von der Dialysewasser-Quelle vor dem Eintritt in das Dialysegerät erheblich erwärmt werden kann. Die Temperaturerhöhung kann beispielsweise der halben Temperaturdifferenz zwischen der Anfangstemperatur des Dialysewassers und der Anfangstemperatur der Dialysatflüssigkeit entsprechen. Mit der Anfangstemperatur ist jeweils die Temperatur der Flüssigkeit gemeint, bevor sie in die Wärmetauschermanschette einläuft.

Ferner bündelt die Wärmetauschermanschette die Ablaufleitung und die Zulaufleitung, so dass die Sichtbarkeit der Leitungen hierdurch verbessert ist und die Anzahl der Stolperfallen reduziert ist. Ferner stellt die Wärmetauschermanschette einen zusätzlichen mechanischen Schutz für die Zulaufleitung und die Ablaufleitung gegen mechanische Beschädigung dar. Die Wärmetauschermanschette einschließlich der Leitungen ist besonders bevorzugt an der Geräterückseite angeordnet.

Vorzugsweise weist die Wärmetauschermanschette einen Isolationskörper auf, der den sich über die Länge erstreckenden Leitungs-Hohlraum aufweist, wobei der Leitungs-Hohlraum bevorzugt mittig im Querschnitt des Isolationskörpers angeordnet ist. Der Isolationskörper weist eine geringere Wärmeleitfähigkeit auf, als der Leitungskörper, der die Zulaufleitung bzw. die Ablaufleistung bildet. Der Isolationskörper besteht besonders bevorzugt aus einem geschäumten Kunststoff.

Der Isolationskörper reduziert die Wärmeverluste der Ablaufleitung bzw. der Zulaufleitung auf ein Minimum. Der Isolationskörper ist im Querschnitt bevorzugt ungefähr kreisrund ausgebildet und weist einen Außendurchmesser von mehreren Zentimetern auf. Der Isolationskörper kann elastisch verformbar sein und kann beispielsweise aus Schaumstoff bestehen, der bei sehr geringen Materialkosten eine hohe Isolationsqualität aufweist.

Gemäß einer bevorzugten Ausgestaltung der Erfindung weist die Wärmetauschermanschette einen separaten Manschettenmantel auf, der den Isolationskörper außen umgibt bzw. umschließt. Der Manschettenmantel besteht aus einem anderen Material als der Isolationskörper. Der Manschettenmantel kann beispielsweise aus einer Kunststoff-Folie bestehen und besonders bevorzugt wärmereflektierend beschichtet sein.

Vorzugsweise weist die Wärmetauschermanschette einen annähernd radialen Öffnungsschlitz auf, der sich in Längsrichtung über die gesamte Länge der Wärmetauschermanschette und in radialer Richtung vom Manschettenrand bis zum Leitungs-Hohlraum erstreckt. Der Öffnungsschlitz schafft also einen lateralen Zugang zu dem Leitungs-Hohlraum. Durch den Öffnungsschlitz wird es ermöglicht, die Wärmetauschermanschette nachträglich an die beiden Leitungen zu applizierten bzw. nach einer Hämodialysebehandlung von den beiden Leitungen zu entfernen. Die Wärmetauschermanschette kann auf diese Weise nachträglich an den beiden Leitungen appliziert werden, bzw. kann entfernt und für mehrere Hämodialysebehandlungen verwendet werden.

Alternativ hierzu kann die Wärmetauschermanschette auch nichtentfernbar ausgebildet sein, und zusammen mit der Zulaufleitung und der Ablaufleitung als Einmalartikel ausgebildet sein. In diesem Fall erfordert sie keinen Öffnungsschlitz.

Gemäß einer bevorzugten Ausgestaltung weist der Leitungs-Hohlraum zwei sich einander überschneidende und im Querschnitt annähernd kreisförmige Teil-Hohlräume auf, wobei der Innendurchmesser des jeweiligen Hohlraums ungefähr annähernd dem Außendurchmesser der betreffenden eingelegten Leitung entspricht. Die Wärmetauschermanschette bzw. der Isolationskörper liegt also dicht bzw. spaltfrei an der Ablaufleitung und der Zulaufleitung an, so dass die beiden Leitungen aufeinander gedrückt werden und unmittelbaren Kontakt haben. Hierdurch wird der Wärmeaustausch zwischen den beiden Leitungen verbessert. Wenn drei Leitungen vorhanden und zu bündeln sind, können auch drei annähernd kreisförmige Teil-Hohlräume vorgesehen sein.

Vorzugsweise weist die Wärmetauschermanschette ein Verschlussmittel zum Verschließen des Öffnungsschlitzes auf. Das Verschlussmittel erlaubt ein einmaliges oder mehrmaliges Verschließen bzw. Öffnen des Öffnungsschlitzes. Das Verschlussmittel kann beispielsweise ein Klebestreifen sein, der sich in Längsrichtung des Öffnungsschlitzes erstreckt, kann aber auch als Klettverschluss-Streifen ausgebildet sein.

Gemäß einer bevorzugten Ausgestaltung weist der Manschettenmantel eine sich in Längsrichtung der Wärmetauschermanschette erstreckende Materialschwächung auf, die eine Sollrisslinie definiert, entlang der der Manschettenmantel aufgerissen werden kann. Die Materialschwächung ist insbesondere für eine als Einmalartikel ausgebildete Wärmetauschermanschette geeignet. Die Materialschwächung kann beispielsweise in Form einer in Längsrichtung verlaufenden Perforation ausgebildet sein. Nach einer Hämodialysebehandlung kann die Wärmetauschermanschette einfach von den Leitungen entfernt werden, indem der Manschettenmantel entlang der Materialschwächung, die bevorzugt radial mit dem Öffnungsschlitz ausgerichtet ist, aufgerissen wird. Die Wärmetauschermanschette kann entfernt werden, und kann beispielsweise getrennt von den Leitungen entsorgt werden.

Vorzugsweise beträgt die Länge der Wärmetauschermanschette mindestens 100 cm, besonders bevorzugt mindestens 150 cm. Bei einer Wärmetauschermanschette von mehr als 100 cm ist die gesamte Kontaktfläche zwischen den beiden Leitungen schon relativ groß, so dass das Dialysewasser in der Zulaufleitung schon einen beträchtlichen Temperaturhub erfahren kann.

Gemäß einer bevorzugten Ausgestaltung ist eine elektrische Energieversorgungsleitung zur Versorgung des Dialysegeräts mit elektrischer Energie vorgesehen. Die Energieversorgungsleitung ist im wesentlichen parallel zu der Zulaufleitung und der Ablaufleitung in dem Leitung-Hohlraum angeordnet. Die Energieversorgungsleitung ist auf diese Weise zum Einen mechanisch geschützt und stellt keine zusätzliche Stolperfalle dar. Grundsätzlich erwärmt sich die Energieversorgungsleitung, sobald das dieser das Dialysegerät elektrische Energie verbraucht. Diese Verlustwärme wird innerhalb der Wärmetauschermanschette zum Erwärmen der Zulaufleitung bzw. des Dialysewassers darin genutzt.

Vorzugsweise ist ein einziger Leitungskörper vorgesehen, der sowohl die Zulaufleitung als auch die Ablaufleitung bildet. Der Leitungskörper kann beispielsweise aus einem geeigneten Kunststoff bestehen. In einem derartigen Leitungskörper kann der Wärmeaustausch zwischen der Zulaufleitung und der Ablaufleitung optimiert werden, beispielsweise, indem die die beiden Leitungen unmittelbar trennende Trennwand in Querrichtung möglichst lang und möglichst dünnwandig ausgeführt ist, um die Wärmeisolation zwischen den beiden Leitungen in diesem Bereich zu minimieren und den Wärmeaustausch zu optimieren.

Im Folgenden werden unter Bezugnahme auf die Zeichnungen Ausführungsbeispiele der Erfindung näher erläutert. Es zeigen:
Figur 1 eine schematische Darstellung einer erfindungsgemäßen Hämodialyseanordnung mit einem Dialysegerät, einer Zulaufleitung, einer Ablaufleitung und einer ersten Ausführungsform einer Wärmetauschermanschette, und
Figur 2 einen Querschnitt der Wärmetauschermanschette der Figur 1 einschließlich der Zulaufleitung und der Ablaufleitung,
Figur 3 einen Querschnitt einer zweiten Ausführungsform einer Wärmetauschermanschette, und
Figur 4 eine ein drittes Ausführungsbeispiel einer Wärmetauschermanschette.

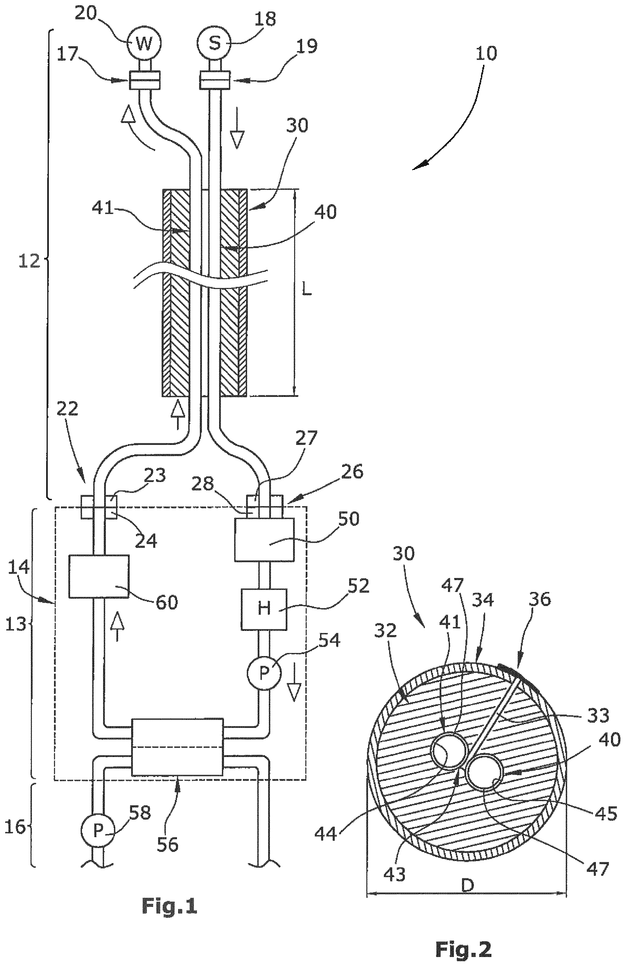

In der Figur 1 ist schematisch eine Hämodialyseanordnung 10 dargestellt, die fluidisch vorliegend in drei Bereiche aufgeteilt ist, nämlich einen Versorgungsbereich 12 mit einer Dialysewasser-Zulaufleitung 40, einer Dialysatflüssigkeits-Ablaufleitung 41 und einer Wärmetauschermanschette 30, einem Bereich 13 mit einem Dialysegerät 14 mit einer Dialyseflüssigkeits-Pumpe 54 und einem Dialysator 56, und einen Blutleitungs-Bereich 16 mit einer Blutpumpe 58, die Blut von einem Patienten durch eine Blutkammer des Dialysators 56 und wieder zurück zu dem Patienten pumpt.

Von einer Dialysewasser-Quelle 18 fließt Dialysewasser, das beispielsweise feinfiltriertes Trinkwasser aus dem öffentlichen Wassernetz sein kann, über eine Leitungskupplung 19 durch die Zulaufleitung 40 zu dem Dialysegerät 14. Die Zulaufleitung 40 weist an ihrem fluidischen Ende ein Kupplungsteil 27 auf, das mit einem geräteseitigen Kupplungsteil 28 lösbar verkuppelt ist. Die beiden Kupplungsteile 27,28 bilden zusammen eine Kupplungsanordnung 26. In dem Dialysegerät 14 fließt das Dialysewasser in einen Dialyseflüssigkeits- Generator 50, in dem aus dem Dialysewasser durch Beimischen entsprechender Substanzen eine geeignete Dialyseflüssigkeit erzeugt wird. Von dem Dialyseflüssigkeits-Generator 50 fließt die Dialyseflüssigkeit zu einer Dialyseflüssigkeits-Heizung 52, in der die Dialyseflüssigkeit auf eine Temperatur von ungefähr 37 °C erwärmt wird. Die Dialyseflüssigkeits-Heizung 52 kann als eine elektrische Heizvorrichtung ausgebildet sein.

Die Dialyseflüssigkeit wird durch eine Pumpe 54 zu dem Dialysator 56 gepumpt, wo harnpflichtigen Substanzen aus dem Patientenblut in die Dialyseflüssigkeit gelangen, die hierdurch begrifflich zu einer Dialysatflüssigkeit wird.

In dem Blutleitungs-Bereich 16 sorgt die Blutpumpe 58 dafür, dass über eine erste Blutleitung Patientenblut von dem Patienten zu der Blutseite des Dialysators 56 gepumpt wird, von wo aus das Patientenblut durch eine zweite Blutleitung zurück zu dem Patienten gepumpt wird.

Die Dialysatflüssigkeit fließt von dem Dialysator 56 aus zu einer Ultrafiltrationseinheit 60, die aus einer oder mehreren Pumpen bestehen kann. Die Ultrafiltrationseinheit 60 bestimmt die Flüssigkeitsbilanz auf der Blutseite bzw. der Dialysatseite des Dialysators 56. Von der Ultrafiltrationseinheit 60 aus fließt das Patientenblut durch eine zweite Steckeranordnung 22 in die Ablaufleitung 41, die in ein Abfallwasser-Ziel 20 mündet. Das Abfallwasser-Ziel 20 kann ein Abfallwasser-Ziel-Behälter sein. Die Ablaufleitung 41 ist über eine entsprechende Leitungskupplung 17 fluidisch mit dem Abfallwasser-Ziel 20 verbunden.

Die Zulaufleitung 40 und die Ablaufleitung 41 werden jeweils durch einen separaten ringförmigen Kunststoff-Leitungskörper 47 gebildet. Der größte Teil der Länge der Zulaufleitung 40 und der Ablaufleitung 41 sind durch eine Wärmetauschermanschette 30 zusammengefasst, die in der Figur 1 schematisch im Längsschnitt und in der Figur 2 im Querschnitt dargestellt ist. Die Wärmetauschermanschette 30 weist einen elastischen und gut wärmeisolierenden Isolationskörper 32 auf, der einen Durchmesser D von mehreren Zentimetern haben kann. Der Isolationskörper 32 besteht aus einem geschäumten Kunststoff.

Wie in der Figur 2 erkennbar ist, weist der außenseitig ungefähr zylindrisch ausgebildete Isolationskörper 32 mittig einen Leitungs-Hohlraum 43 auf, der aus zwei im Querschnitt annähernd kreisförmigen Teil-Hohlräumen 44,45 gebildet ist, die einen im Querschnitt annähernd 8-förmigen Leitungs-Hohlraum 43 bilden. In den beiden Teil-Hohlräumen 44,45 sind die beiden Leitungen 40,41 unmittelbar aneinander angrenzend oder aneinander anliegend in Längsrichtung eingelegt.

Der Isolationskörper 32 ist zirkulär eingehüllt durch einen Manschettenmantel 34, der von einer Kunststofffolie gebildet wird. Der Manschettenmantel 34 ist bevorzugt wärmereflektierend ausgebildet.

Von dem Leitungs-Hohlraum 43 aus erstreckt sich ein radialer Öffnungsschlitz 33 nach außen, der sowohl den Isolationskörper 32 zur Hälfte als auch den Manschettenmantel 34 durchdringt, so dass nach Öffnen eines sich über die gesamte Längserstreckung des Öffnungsschlitzes 33 erstreckenden Verschlussmittels 36 die Wärmetauschermanschette 30 aufgeklappt und von den Leitungen 40,41 entfernt werden kann.

Das Verschlussmittel 36 ist vorliegend als Klettverschluss ausgebildet, kann alternativ jedoch auch als lösbarer Klebeverschluss ausgebildet sein.

In einer alternativen Ausgestaltung ist kein Verschlussmittel vorgesehen, sondern ist statt des Öffnungsschlitzes 33 nur eine Materialschwächung in Form einer sich in Längsrichtung erstreckenden Perforation vorgesehen. Nach Abschluss der Hämodialysebehandlung kann der Manschettenmantel entlang der Materialschwächung einfach aufgerissen werden und die Wärmetauschermanschette von den Leitungen 41,41 entfernt und gesondert von diesen entsorgt werden.

Die Wärmetauschermanschette 30 hat eine Länge von ca. 150 cm. Bei einer nachrüstbaren Wärmetauschermanschette ist auch denkbar, dass diese eine Länge von mehr als 150 cm hat, und vor Ort auf die erforderliche Länge zugeschnitten wird.

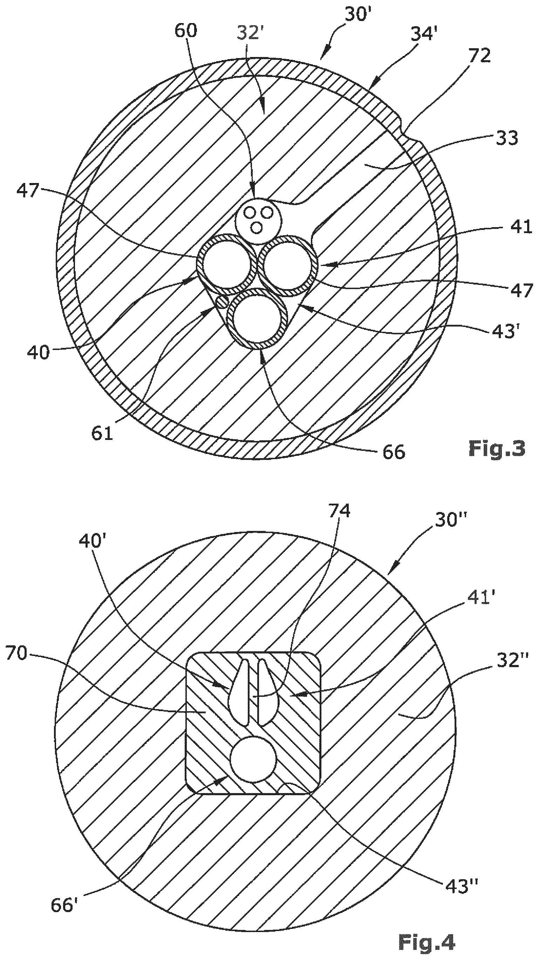

In der Figur 3 ist ein zweites Ausführungsbeispiel einer Wärmetauschermanschette 30' dargestellt. Im Unterschied zu der Wärmetauschermanschette 30 der Figuren 1 und 2 weist die Wärmetauschermanschette 30' der Figur 3 einen größeren Leitungs-Hohlraum 43' auf. In dem von dem Isolationskörper 32' definierten Leitung-Hohlraum 43' sind neben der Zulaufleitung 40 und der Ablaufleitung 41 eine weitere Flüssigkeitsleitung 66, eine Energieversorgungsleitung 60 sowie eine Schutzerdungs-Leitung 61 parallel zueinander und miteinander gebündelt angeordnet. Die Flüssigkeitsleitung 66 kann beispielsweise eine Konzentrat-Leitung sein, eine Bikarbonat-Leitung sein oder eine andere Flüssigkeitsleitung sein, die eine für die Dialyse in dem Dialysegerät 14 benötigte Flüssigkeit transportiert.

Die Wärmetauschermanschette 30' weist einen Manschettenmantel 34' auf, der in radialer Ausrichtung mit dem radialen Öffnungsschlitz 33 eine in Längsrichtung verlaufende Materialschwächung 72 aufweist, die vorliegend als radial nicht-geöffnete durchgehende Nut mit in Längsrichtung durchgehendem Nutboden ausgebildet ist. Grundsätzlich kann die Materialschwächung aber auch als Perforations-Linie oder auf andere Weise ausgebildet sein. Sobald die Wärmetauschermanschette 30' nicht mehr benötigt wird, kann sie über die ganze Länge im Bereich der Materialschwächung 72 aufgerissen und geöffnet werden, und von den Bündel der Leitungen 40,41,60,66,61 abgenommen und entfernt werden.

In der Figur 4 ist ein drittes Ausführungsbeispiel einer Wärmetauschermanschette 30" dargestellt. Die Wärmetauschermanschette 30" besteht aus einem Isolationskörper 32", der in Umfangsrichtung jedoch geschlossen ausgebildet ist, also keinen radialen Öffnungsschlitz aufweist. Ferner weist die Wärmetauschermanschette 30", im Gegensatz zu den ersten beiden Ausführungsbeispielen, keinen Manschettenmantel auf.

In dem Leitungs-Hohlraum 43" ist ein einziger Kunststoff-Leitungskörper 70 angeordnet, der seinerseits über entsprechende und sich über die gesamte Länge erstreckende Hohlräume verfügt, durch die die Zulaufleitung 40', die Ablaufleitung 41' sowie eine weitere Flüssigkeitsleitung 66' gebildet sind.

Die Zulaufleitung 40' und die Ablaufleitung 41' sind im Querschnitt jeweils ungefähr halbkreisförmig ausgebildet, wobei die ebenen Leitungswand-Oberflächen der beiden Leitungen 40',41' von einer einzigen Trennwand 74 gebildet werden, die relativ dünn ist. Auf diese Weise ist der Wärmeaustausch zwischen der Zulaufleitung 40' und der Ablaufleitung 41' verbessert und erleichtert.

## Patentansprüche

1. Hämodialyseanordnung (10) mit
einer Dialysewasser-Zulaufleitung (40) gebildet aus einem Kunststoff-Leitungskörper (47; 70), durch die Dialysewasser aus einer Dialysewasser-Quelle (18) zu einem Dialysegerät (14) fließt,
einer Dialysat-Ablaufleitung (41) gebildet aus einem Kunststoff-Leitungskörper (47; 70), durch die Dialysatflüssigkeit von dem Dialysegerät (14) zu einem Abfallwasser-Ziel (20) fließt, und
einer langgestreckten Hämodialyse-Wärmetauschermanschette (30,30',30"), die einen sich in Längsrichtung der Wärmetauschermanschette (30;30';30") erstreckenden Leitungs-Hohlraum (43;43';43") aufweist, in dem die Zulaufleitung (40) und die Ablaufleitung (41) im Wesentlichen parallel zueinander im Gegenstrom und benachbart zueinander eingebettet sind.

2. Hämodialyseanordnung (10) nach Anspruch 1, wobei die Wärmetauschermanschette (30;30';30") einen separaten Isolationskörper (32;32';32") aufweist, der den sich in Flussrichtung erstreckenden Leitungs-Hohlraum (43;43;43") aufweist und der eine geringere Wärmeleitfähigkeit aufweist, als der Leitungskörper (47;70), der die Zulaufleitung (40) und die Ablaufleitung (41) bildet.

3. Hämodialyseanordnung (10) nach Anspruch 2, wobei die Wärmetauschermanschette (30;30') einen separaten Manschettenmantel (34) aufweist, der den Isolationskörper (32;32') umgibt.

4. Hämodialyseanordnung (10) nach einem der vorangegangenen Ansprüche 2 oder 3, wobei die Wärmetauschermanschette (30;30') einen annähernd radialen Öffnungsschlitz (33) aufweist, der sich in Längsrichtung über die gesamte Länge (L) der Wärmetauschermanschette (30;30") und in radialer Richtung vom Manschettenrand bis zum Leitungs-Hohlraum (43;43') erstreckt.

5. Hämodialyseanordnung (10) nach einem der vorangegangenen Ansprüche, wobei der Leitungs-Hohlraum (43) zwei sich einander überschneidende und im Querschnitt annähernd kreisförmige Teil-Hohlräume (44,45) aufweist, wobei der Innendurchmesser des jeweiligen Hohlraums (44,45) jeweils annähernd dem Außendurchmesser der eingelegten Leitung (41,40) entspricht.

6. Hämodialyseanordnung (10) nach Anspruch 4, wobei die Wärmetauschermanschette (30) ein wiederverschließbares Verschlussmittel (36) zum Verschließen des Öffnungsschlitzes (33) aufweist.

7. Hämodialyseanordnung (10) nach Anspruch 3 oder nach einem der Ansprüche 4 oder 5 wenn sie von Anspruch 3 abhängig sind, wobei der Manschettenmantel (34') eine in Längsrichtung verlaufende Materialschwächung (72) aufweist, die eine Sollrisslinie definiert, entlang der der Manschettenmantel (34') aufgerissen werden kann.

8. Hämodialyseanordnung (10) nach einem der vorangegangenen Ansprüche, wobei die Länge (L) der Wärmetauschermanschette (30;30';30") mindestens 100 cm, besonders bevorzugt mindestens 150 cm beträgt.

9. Hämodialyseanordnung (10) nach einem der vorangegangenen Ansprüche, wobei eine elektrische. Energieversorgungsleitung (60) vorgesehen ist, die in dem Leitungs-Hohlraum (43") im Wesentlichen parallel zu der Zulaufleitung (40) und der Ablaufleitung (41) angeordnet ist.

10. Hämodialyseanordnung (10) nach einem der vorangegangenen Ansprüche, wobei die Zulaufleitung (40') und die Ablaufleitung (41') von einem einzigen Leitungskörper (70) gebildet ist.

## Claims

1. Hemodialysis arrangement (10) comprising
a dialysis water supply line (40) formed by a plastic material line body (47; 70), via which line dialysis water flows from a dialysis water source (18) to a dialysis apparatus (14),
a dialysate drain line (41) formed by a plastic material line body (47; 70), via which line dialysate liquid flows from the dialysis apparatus (14) to a waste water target (20), and
an elongated hemodialysis heat exchanger sleeve (30, 30', 30"), which extends in a longitudinal direction of the heat exchanger sleeve (30; 30'; 30") having a line cavity (43; 43'; 43") extending in the longitudinal direction of the heat exchanger sleeve (30; 30'; 30"), in which the inlet line (40) and the drain line (41) are embedded essentially parallel to one another in counter-flow and adjacent to one another.

2. Hemodialysis arrangement (10) of claim 1, wherein the heat exchanger sleeve (30; 30'; 30") comprises a separate insulation body (32; 32'; 32") which includes the conduit cavity (43; 43'; 43") extending in the direction of flow and which has a lower thermal conductivity than the line body (47; 70) which forms the inlet line (40) and the drain line (41).

3. Hemodialysis arrangement (10) of claim 2, wherein the heat exchanger sleeve (30; 30') comprises a separate sleeve jacket (34) which surrounds the insulation body (32; 32').

4. Hemodialysis arrangement (10) of one of the preceding claims 2 or 3, wherein the heat exchanger sleeve (30; 30') comprises an approximately radial opening slot (33) which extends in the longitudinal direction over the entire length (L) of the heat exchanger sleeve (30; 30') and extends in the radial direction from the sleeve edge to the line cavity (43; 43').

5. Hemodialysis arrangement (10) of one of the preceding claims, wherein the line cavity (43) comprises two partial cavities (44,45) that overlap each other and are approximately circular in cross-section, the inner diameter of the respective cavity (44,45) approximately corresponding to the outside diameter of the inserted line (41,40).

6. Hemodialysis arrangement (10) of claim 4, wherein the heat exchanger sleeve (30) has a resealable closure means (36) for closing the opening slot (33).

7. Hemodialysis arrangement (10) of claim 3 or one of claims 4 or 5, if dependent on claim 3, wherein the sleeve jacket (34') has a longitudinally extending material weakening (72) which defines a predetermined tear line along which the sleeve jacket (34') can be torn open.

8. Hemodialysis arrangement (10) of one of the preceding claims, wherein the length (L) of the heat exchanger sleeve (30; 30'; 30") is at least 100 cm, particularly preferably at least 150 cm.

9. Hemodialysis arrangement (10) of one of the preceding claims, wherein an electrical power supply line (60) is provided, which is arranged in the line cavity (43') substantially parallel to the inlet line (40) and the drain line (41).

10. Hemodialysis arrangement (10) of one of the preceding claims, wherein the inlet line (40') and the drain line (41') are formed by a single line body (70).

## Revendications

1. Dispositif d'hémodialyse (10) comprenant
une conduite d'alimentation en eau de dialyse (40), formée par un corps de conduite (47; 70) en matière plastique, à travers laquelle l'eau de dialyse s'écoule d'une source d'eau de dialyse (18) vers un appareil de dialyse (14),
une conduite de drainage de dialysat (41), formée par un corps de conduite (47; 70) en matière plastique, à travers laquelle le fluide de dialysat s'écoule de l'appareil de dialyse (14) vers une destination d'eaux usées (20), et
un manchon d'échangeur de chaleur d'hémodialyse (30, 30', 30") allongé, comprenant une cavité de conduite (43; 43'; 43") qui s'étend dans la direction longitudinale du manchon d'échangeur de chaleur (30; 30'; 30"), dans laquelle la conduite d'entrée (40) et la conduite de drainage (41) sont noyées essentiellement parallèlement l'une à l'autre en contre-courant et adjacentes l'une à l'autre.

2. Dispositif d'hémodialyse (10) selon la revendication 1, dans lequel le manchon d'échangeur de chaleur (30; 30'; 30") comprend un corps d'isolation séparé (32; 32'; 32") comprenant la cavité de conduite (43; 43; 43") s'étendant dans le sens de l'écoulement, et qui comprend une conductivité thermique inférieure à celle du corps de conduite (47; 70) qui forme la conduite d'entrée (40) et la conduite de drainage (41).

3. Dispositif d'hémodialyse (10) selon la revendication 2, dans lequel le manchon d'échangeur de chaleur (30; 30') comprend une chemise de manchon séparée (34) qui entoure le corps isolant (32; 32').

4. Dispositif d'hémodialyse (10) selon l'une quelconque des revendications 2 ou 3 précédentes, dans lequel le manchon d'échangeur de chaleur (30; 30') comprend une fente d'ouverture (33) approximativement radiale qui s'étend dans le sens longitudinal sur toute la longueur (L) du manchon d'échangeur de chaleur (30; 30') et s'étend dans la direction radiale depuis le bord du manchon jusqu'à la cavité de conduite (43; 43').

5. Agencement d'hémodialyse (10) selon l'une quelconque des revendications précédentes, dans lequel la cavité de conduite (43) comprend deux cavités partielles se chevauchant et à section approximativement circulaire (44, 45), le diamètre intérieur de la cavité (44, 45) respective correspondant approximativement au diamètre extérieur de la conduite insérée (41.40).

6. Dispositif d'hémodialyse (10) selon la revendication 4, dans lequel le manchon d'échangeur de chaleur (30) comprend un moyen de fermeture refermable (36) pour fermer la fente d'ouverture (33).

7. Dispositif d'hémodialyse (10) selon la revendication 3 ou une des revendications 4 à 5, si dépendant de la revendication 3, dans lequel la chemise de manchon (34') présente un affaiblissement (72) longitudinal qui définit une ligne de déchirure prédéterminée, le long de laquelle la chemise de manchon (34') peut être déchirée.

8. Dispositif d'hémodialyse (10) selon l'une quelconque des revendications précédentes, dans lequel la longueur (L) du manchon d'échangeur de chaleur (30; 30'; 30") est d'au moins 100 cm, de manière particulièrement préférée d'au moins 150 cm.

9. Dispositif d'hémodialyse (10) selon l'une quelconque des revendications précédentes, dans lequel une conduite d'alimentation électrique (60) est prévue, qui est disposée dans la cavité de conduite (43') sensiblement en parallèle à la conduite d'entrée (40) et la conduite de drainage (41).

10. Dispositif d'hémodialyse (10) selon l'une quelconque des revendications précédentes, dans lequel la conduite d'entrée (40') et la conduite de drainage (41') sont formées par un seul corps de conduite (70).
